# EUROPEAN PATENT SPECIFICATION

(11) **EP 2 377 470 B1**
(45) Date of publication and mention of the grant of the patent: **21.01.2015**
(21) Application number: 11250341.2
(22) Date of filing: 18.03.2011
(51) Int. Cl.: A61B 17/00, A61B 17/06, B60R 13/02, A61B 17/04

(54) **Tapered looped suture**
Verjüngte Schlaufennaht
Suture biseautée à boucle

(30) Priority: 19.03.2010 US 727380
(43) Date of publication of application: 19.10.2011
(60) Divisional application: 14181296.6
(73) Proprietor: Covidien LP, Mansfield, MA 02048 (US)
(72) Inventor: Hadba, Ahmad Robert, Middlefield, CT 06455 (US); Hodgkinson, Gerald, Guilford, CT 06437 (US); Maiorino, Nicholas, Branford, CT 06405 (US); Bowns, William R, Ansonia, CT 06401 (US)
(74) Representative: Soames, Candida Jane

(56) References cited:
- EP-A1- 0 560 135
- EP-A1- 2 163 207
- WO-A2-03/088818
- US-A- 5 002 563
- US-A1- 2003 191 479
- US-A1- 2010 204 730
- LENDLEIN A ET AL: "Biodegradable, elastic shape-memory polymers for potential biomedical applications", SCIENCE, AMERICAN ASSOCIATION FOR THE ADVANCEMENT OF SCIENCE, WASHINGTON, DC; US, vol. 296, no. 5573, 31 May 2002 (2002-05-31), pages 1673-1676, XP002516073, ISSN: 0036-8075, DOI: DOI:10.1126/SCIENCE.1066102 [retrieved on 2002-04-25]

## Description

### BACKGROUND

### Technical Field

The present disclosure relates to a suture having a loop. The looped suture may have a taper cut.

### Background of Related Art

Sutures including loops formed therein are known. A loop formed in a suture during manufacture may be used to secure the suture to tissue. In this manner, once the non-looped end of the suture is inserted through tissue, that end may be threaded through the loop to form a slip knot-like configuration that may be tied to secure tissue. In another application, a loop may be formed in a suture in place of a knot. This requires the use of a handheld instrument that may be brought into an operating room.

Regardless of the reason for forming the loop, when a loop is formed in a suture, whether using adhesive, heat or ultrasonic energy, the diameter of the suture is doubled where the two suture portions overlap. In the event that the suture loop is used to secure tissue, the doubling of the diameter of the suture in order to create the loop may increase the amount of force necessary to pull the loop through tissue. This increased force applied to the suture may result in tearing or other unnecessary trauma to the tissue being sutured. Therefore, it would be beneficial to have a looped suture to include a taper cut and methods of making such a suture.

WO 03/088818 A2 relates to shape memory polymeric devices and more particularly to shape memory polymeric sutures. WO 03/088818 A2 discloses a suture capable of forming a self-tightening knot. A method of closing a wound or body scission is disclosed which generally includes the steps of (1) providing a suture formed of a shape memory polymer; (2) forming a loose knot of the suture; and (3) heating the suture to close the wound or body scission.

### SUMMARY

The present invention is defined in independent claim 1 and certain optional features thereof are defined in the dependent claims.

A looped suture is provided. The suture includes an elongate body including a proximal end and a distal end and a loop integrally formed on the distal end of the elongate body. At least a portion of the loop includes a shape memory polymeric material. The shape memory polymeric material is configured to radially expand and axially shorten when transitioning from a temporary configuration to a permanent configuration. The radial expansion and axial shortening causes constricting of the loop.

In embodiments, the loop of the suture is substantially bulbous when the shape memory polymeric material is in a temporary configuration. In embodiments, the loop is substantially flattened when the shape memory polymeric material transitions from the temporary configuration to the permanent configuration.

Any one of the presently disclosed embodiments may further include a plurality of surface features such as barbs, hooks, latches, protrusions, leaves, teeth, and/or combinations thereof. The surface feature may include a shape memory polymer.

There is described a method of using a looped suture. The method includes providing a suture including an elongate body and a loop formed on a distal end of the elongate body, wherein at least a portion of the loop is formed of a shape memory polymeric material; inserting a proximal end of the elongate body into tissue; pulling the elongate body through the tissue; receiving the proximal end of the elongated body through the loop; and effecting the transition of the shape memory polymeric material.

### BRIEF DESCRIPTION OF THE DRAWINGS

The accompanying drawings, which are incorporated in and constitute a part of this specification, illustrate embodiments of the disclosure and, together with a general description of the disclosure given above, and the detailed description of the embodiment(s) given below, serve to explain the principles of the disclosure, wherein:

FIG. 1 is a side view of a looped suture in accordance with one embodiment of the present disclosure;

FIG. 1A is a side view of a looped suture in accordance with another embodiment of the present disclosure;

FIG. 2 is a cross-sectional end view of the looped suture taken along line 2-2 of FIG. 1;

FIG. 3 is an enlarged side view of portion 3 of FIG. 1;

FIGS. 4A-4F are perspective views of a thread having a circular (FIG. 4A), oval (FIG. 4B), rectangular (square) (FIG. 4C), flat (FIG. 4D), octagonal (FIG. 4E), and rectangular (FIG. 4F) cross-sectional profiles;

FIG. 5A-5C are views of an alternate embodiment of a looped suture of the present disclosure;

FIG. 6A-6C are views of yet another embodiment of a looped suture of the present disclosure;

FIGS. 7A-7C are views of still another embodiment of a looped suture of the present disclosure;

FIGS. 8-8B are side views of a looped suture in accordance with yet another embodiment of the present disclosure in a first configuration (FIG. 8) and in second configurations (FIGS. 8A and 8B); and

FIGS. 9-9B are side views of a looped suture in accordance with yet another embodiment of the present disclosure in a first configuration (FIG. 9) and in second configurations (FIGS. 9A and 9B).

### DETAILED DESCRIPTION

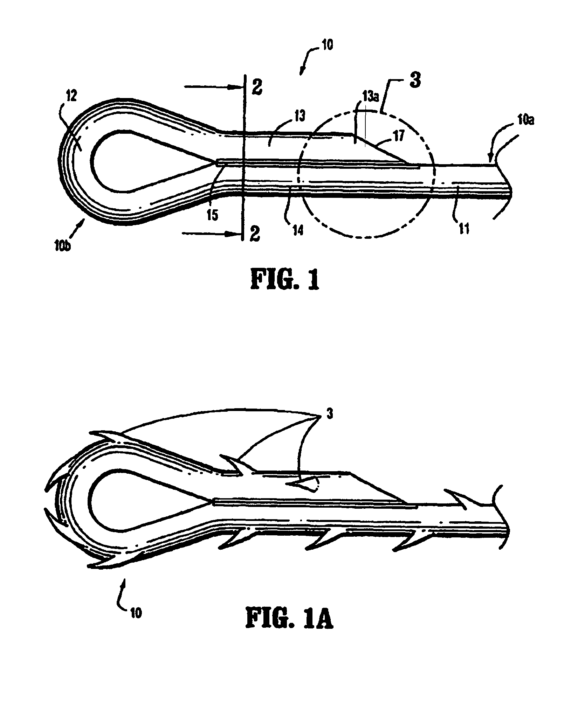

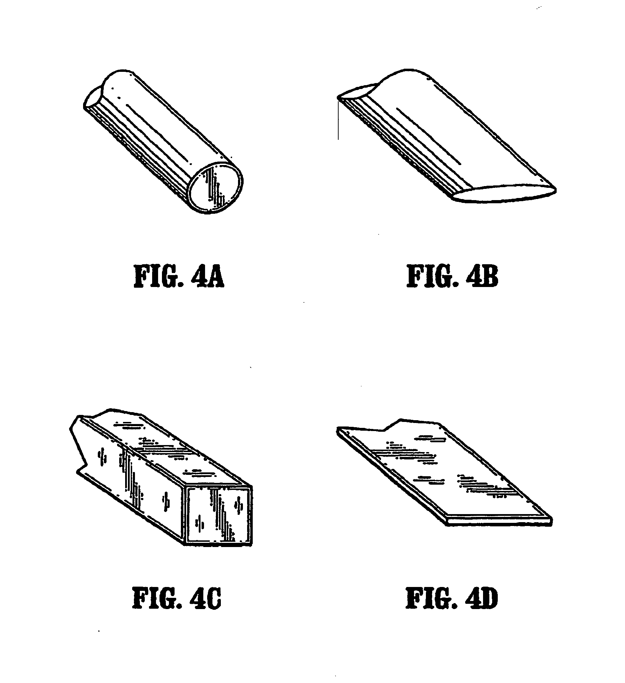

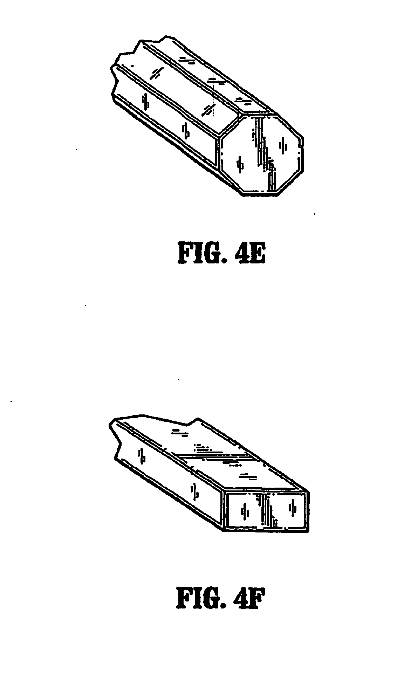

As shown in FIG. 1, an embodiment of a suture according to the present disclosure is shown generally as looped suture 10. Suture 10 is formed a thread 11 and includes a loop 12 on a distal end 10b thereof. Although shown having a circular cross-sectional geometry, the cross-sectional geometry of thread 11 may be of any suitable shape. For example, FIGS. 4A- 4F illustrate cross-sectional views of alternative embodiments of the various cross-sectional geometries of thread 11, namely, round (FIG. 4A), elliptical (FIG. 4B), square (FIG.4C), flat (FIG. 4D), octagonal (FIG.4E), and rectangular (FIG. 4F).

Thread 11 may be formed of any material within the purview of those skilled in the art, such as, for example, degradable materials, non-degradable materials, natural materials, synthetic materials, shape memory materials, metals, alloys, and combinations thereof.

More particularly, thread 11 may be formed of a degradable material selected from the group consisting of polyesters, polyorthoesters, polymer drugs, polydroxybutyrates, lactones, proteins, cat gut, collagens, carbonates, homopolymers thereof, copolymers thereof, and combinations thereof. In other embodiments, suitable degradable materials which may be utilized to form thread 11 include natural collagenous materials or synthetic resins including those derived from alkylene carbonates such as trimethylene carbonate, tetramethylene carbonate, and the like; caprolactone; dioxanone; glycolic acid; lactic acid; homopolymers thereof; copolymers thereof; and combinations thereof. In some embodiments, glycolide and lactide based polyesters, especially copolymers of glycolide and lactide, may be utilized to form thread 11.

In yet other embodiments, suitable materials for forming thread 11 include homopolymers, copolymers, and/or blends possessing glycolic acid, lactic acid, glycolide, lactide, dioxanone, trimethylene caprolactone, and various combinations of the foregoing. For example, in some embodiments, a copolymer of glycolide and trimethylene carbonate is used to form thread 11. Methods for forming such copolymers are within the purview of those skilled in the art and include, for example, the methods disclosed in U.S. Patent Nos. 4,300,565 and 5,324,307. Suitable copolymers of glycolide and trimethylene carbonate may possess glycolide in amounts from about 60% to about 75% by weight of the copolymer, in embodiments, from about 65% to about 70% by weight of the copolymer, with the trimethylene carbonate being present in amounts from about 25% to about 40% by weight of the copolymer, in embodiments, from about 30% to about 35% by weight of the copolymer.

Other suitable materials for forming thread 11 include copolymers of lactide and glycolide, with lactide present in an amount from about 6% to about 12% by weight of the copolymer and glycolide being present in amounts from about 88% to about 94% by weight of the copolymer. In some embodiments, lactide is present from about 7% to about 11 % by weight of the copolymer with glycolide being present in amounts from about 89% to about 98% by weight of the copolymer. In some other embodiments, lactide is present in an amount of about 9% by weight of the copolymer with the glycolide being present in an amount of about 91 % by weight of the copolymer.

In some embodiments, suitable materials for forming thread 11 include copolymers of glycolide, dioxanone, and trimethylene carbonate. Such materials may include, for example, copolymers possessing glycolide in amounts from about 55% to about 65% by weight of the copolymer, in embodiments, from about 58% to about 62% by weight of the copolymer, in some embodiments, about 60% by weight of the copolymer; dioxanone in amounts from about 10% to about 18% by weight of the copolymer, in embodiments, from about 12% to about 16% by weight of the copolymer, in some embodiments about 14% by weight of the copolymer; and trimethylene carbonate in amounts from about 17% to about 35% by weight of the copolymer, in embodiments, from about 22% to about 30% by weight of the copolymer, in some embodiments, about 26% by weight of the copolymer.

Other suitable materials for forming thread 11 include a copolymer of glycolide, lactide, trimethylene carbonate, and e-caprolactone. Such materials may include, for example, a random copolymer possessing caprolactone in amounts from about 14% to about 20% by weight of the copolymer, in embodiments, from about 16% to about 18% by weight of the copolymer, in some embodiments, about 17% by weight of the copolymer; lactide in amounts from about 4% to about 10% by weight of the copolymer, in embodiments, from about 6% to about 8% by weight of the copolymer, in some embodiments about 7% by weight of the copolymer; trimethylene carbonate in amounts from about 4% to about 10% by weight of the copolymer, in embodiments from about 6% to about 8% by weight of the copolymer, in some embodiments about 7% by weight of the copolymer; and glycolide in amounts from about 60% to about 78% by weight of the copolymer, in embodiments, from about 66% to about 72% by weight of the copolymer, in some embodiments about 69% by weight of the copolymer.

In other embodiments, thread 11 may be formed of non-degradable materials. Such materials include polyolefins, including polypropylene, polyethylene, and copolymers and blends including same; polytetrafluoroethylene; polyether-esters such as polybutester; silk; cotton; linen; carbon fibers; and the like. The polypropylene may be isotactic polypropylene or a mixture of isotactic and syndiotactic or atactic polypropylene.

In some embodiments, thread 11 is formed of a shape memory material. Shape memory materials possess a permanent shape and a temporary shape. Commonly, the temporary shape is of a configuration which enhances the ability of a surgeon to introduce thread 11 into a patient's body. The permanent shape, which is assumed in vivo upon application of energy, such as heat or light, is of a configuration which enhances the retention of thread 11 in tissue.

Shape memory polymers are a class of polymers that, when formed into an object such as thread 11, can be temporarily deformed by mechanical force and then caused to revert back to an original shape when stimulated by energy. Shape memory polymers exhibit shape memory properties by virtue of at least two phase separated microdomains in their microstructure. The first domain is composed of hard, covalently cross-linked or otherwise chain motion-limiting structures, which act as anchors to retain the object's original shape. The second domain is a switchable soft structure, which can be deformed and then fixed to obtain a secondary or temporary shape.

In the case of heat stimulated shape memory polymers, a transition temperature (T_{Trans}) exists at which the shape change occurs during heating. The shape memory polymers can thus be tailored by altering material properties at the molecular level and by varying processing parameters. An object's primary shape may be formed with heat and pressure at a temperature at which the soft domains are flexible and the hard domains are not fully formed. The object may then be cooled so that the hard domains are more fully formed and the soft domains become rigid. The secondary or temporary shape can be formed by mechanically deforming the object, which is most readily accomplished at a temperature approaching or above T_{Trans}. Mechanical stresses introduced into the object are then locked into place by cooling the object to temperatures below T_{Trans}, so that the soft segments solidify to a rigid state. Once the object is heated to T>T_{Trans}, the soft segments soften and relax back to their original configuration and the object returns to its primary or original shape, sometimes referred to herein, as its permanent shape. The temperature at which a shape memory material reverts to its permanent shape may be referred to, in embodiments, as its permanent temperature (Tₚₑᵣₘ).

Polymers possessing shape memory properties which may be used to construct thread 11 include, for example, synthetic materials, natural materials (e.g., biological) and combinations thereof, which may be biodegradable and/or non-biodegradable. As used herein, the term "biodegradable" includes both bioabsorbable and bioresorbable materials. By "biodegradable", it is meant that the materials decompose, or lose structural integrity under body conditions (e.g., enzymatic degradation, hydrolysis) or are broken down (physically or chemically) under physiologic conditions in the body (e.g., dissolution) such that the degradation products are excretable or absorbable by the body.

Suitable non-degradable materials that may be used to form thread 11 include, but are not limited to, polyolefins such as polyethylene (including ultra high molecular weight polyethylene) and polypropylene including atactic, isotactic, syndiotactic, and blends thereof; polyethylene glycols; polyethylene oxides; ultra high molecular weight polyethylene; copolymers of polyethylene and polypropylene; polyisobutylene and ethylene-alpha olefin copolymers; fluorinated polyolefins such as fluoroethylenes, fluoropropylenes, fluoroPEGs, and polytetrafluoroethylene; polyamides such as nylon, Nylon 6, Nylon 6,6, Nylon 6,10, Nylon 11, Nylon 12, and polycaprolactam; polyamines; polyimines; polyesters such as polyethylene terephthalate, polyethylene naphthalate, polytrimethylene terephthalate, and polybutylene terephthalate; polyethers; polytetramethylene ether glycol; polybutesters, including copolymers of butylene terephthalate and polytetramethylene ether glycol; 1,4-butanediol; polyurethanes; acrylic polymers; methacrylics; vinyl halide polymers and copolymers such as polyvinyl chloride; polyvinyl alcohols; polyvinyl ethers such as polyvinyl methyl ether; polyvinylidene halides such as polyvinylidene fluoride and polyvinylidene chloride; polychlorofluoroethylene; polyacrylonitrile; polyaryletherketones; polyvinyl ketones; polyvinyl aromatics such as polystyrene; polyvinyl esters such as polyvinyl acetate; copolymers of vinyl monomers with each other and olefins such as ethylene-methyl methacrylate copolymers; acrylonitrile-styrene copolymers; ABS resins; ethylene-vinyl acetate copolymers; alkyd resins; polycarbonates; polyoxymethylenes; polyphosphazine; polyimides; epoxy resins; aramids; rayon; rayon-triacetate; spandex; silicones; and copolymers and combinations thereof. Additionally, non-biodegradable polymers and monomers may be combined with each other.

Suitable bioabsorbable polymers for forming thread 11 include, but are not limited to, aliphatic polyesters; polyamides; polyamines; polyalkylene oxalates; poly(anhydrides); polyamidoesters; copoly(ether-esters); poly(carbonates) including tyrosine derived carbonates; poly(hydroxyalkanoates) such as poly(hydroxybutyric acid), poly(hydroxyvaleric acid), and poly(hydroxybutyrate); polyimide carbonates; poly(imino carbonates) such as poly (bisphenol A-iminocarbonate and the like); polyorthoesters; polyoxaesters including those containing amine groups; polyphosphazenes; poly (propylene fumarates); polyurethanes; polymer drugs such as polydiflunisol, polyaspirin, and protein therapeutics; biologically modified (e.g., protein, peptide) bioabsorbable polymers; and copolymers, block copolymers, homopolymers, blends, and combinations thereof.

Suitable aliphatic polyesters for forming thread 11 include, but are not limited to, homopolymers and copolymers of lactide (including lactic acid, D-,L- and meso lactide); glycolide (including glycolic acid); epsilon-caprolactone; p-dioxanone (1,4-dioxan-2-one); trimethylene carbonate (1,3-dioxan-2-one); alkyl derivatives of trimethylene carbonate; Δ -valerolactone; β-butyrolactone; γ-butyrolactone; ε-decalactone; hydroxybutyrate; hydroxyvalerate; 1,4-dioxepan-2-one (including its dimer 1,5,8,12-tetraoxacyclotetradecane-7,14-dione); 1,5-dioxepan-2-one; 6,6-dimethyl- 1,4-dioxan-2-one; 2,5-diketomorpholine; pivalolactone; α, α diethylpropiolactone; ethylene carbonate; ethylene oxalate; 3-methyl-1,4-dioxane-2,5-dione; 3,3-diethyl-1,4-dioxan-2,5-dione; 6,8-dioxabicycloctane-7-one; and polymer blends and copolymers thereof.

Other suitable biodegradable polymers for forming thread 11 include, but are not limited to, poly(amino acids) including proteins such as collagen (I, II and III), elastin, fibrin, fibrinogen, silk, and albumin; peptides including sequences for laminin and fibronectin (RGD); polysaccharides such as hyaluronic acid (HA), dextran, alginate, chitin, chitosan, and cellulose; glycosaminoglycan; gut; and combinations thereof. Collagen as used herein includes natural collagen such as animal derived collagen, gelatinized collagen, or synthetic collagen such as human or bacterial recombinant collagen.

Additionally, synthetically modified natural polymers such as cellulose and polysaccharide derivatives, including alkyl celluloses, hydroxyalkyl celluloses, cellulose ethers, cellulose esters, nitrocelluloses, and chitosan may be utilized to form thread 11. Examples of suitable cellulose derivatives include methyl cellulose, ethyl cellulose, hydroxypropyl cellulose, hydroxypropyl methyl cellulose, hydroxybutyl methyl cellulose, cellulose acetate, cellulose propionate, cellulose acetate butyrate, cellulose acetate phthalate, carboxymethyl cellulose (CMC), cellulose triacetate, and cellulose sulfate sodium salt. These may be collectively referred to herein, in embodiments, as "celluloses."

In some embodiments, thread 11 may include combinations of both degradable and non-degradable materials. The degradable and/or non-degradable materials used may have shape memory characteristics.

In other embodiments, the shape memory polymer forming thread 11 is a copolymer of two components with different thermal characteristics, such as oligo (epsilon-caprolactone) dimethacrylates and butyl acrylates, including poly(epsilon-caprolactone) dimethacrylate-poly (n-butyl acrylate), or a diol ester and an ether-ester diol such as oligo (epsilon caprolactone) diol/oligo (p-dioxanone) diol copolymers. These multi-block oligo (epsilon-caprolactone) diol/oligo (p-dioxanone) diol copolymers possess two block segments: a "hard" segment and a "switching" segment linked together in linear chains. Such materials are disclosed, for example, in Lendlein, "Shape Memory Polymers-Biodegradable Sutures," Materials World, Vol. 10, no. 7, pp. 29-30 (July 2002).

In still other embodiments, thread 11 is formed of blends of bioabsorbable materials including, but not limited to, urethanes blended with lactic acid and/or glycolic acid, homopolymers thereof or copolymers thereof, and acrylates blended with caprolactones such as polycaprolactone dimethacrylate poly(butyl acrylate) blends, and combinations thereof.

Other examples of suitable shape memory polymers and means for forming permanent and temporary shapes therewith are set forth in Lendlein et al., "Shape memory polymers as stimuli-sensitive implant materials," Clinical Hemorheology and Microcirculation, 32 (2005) 105-116, Lendlein et al., "Biodegradable, Elastic Shape memory Polymers for Potential Biomedical Applications," Science, Vol. 269 (2002) 1673-1676, and Lendlein et al., "Shape-Memory Polymers," Angew. Chem. Int. Ed., 41 (2002) 2035-2057.

Table 1 below further illustrates compositions which demonstrate shape memory effects and may be used to form thread 11. The block copolymers of each composition are in annealed wire format, the proposed soft and hard segments, and the glass transition temperature (Tg), having been measured by differential scanning calorimetry which is equal to T_{Trans}.

**TABLE 1**

| Composition (mol%) | Soft Domain | Hard Domain | T_{g} (T_{Trans}) |
|---|---|---|---|
| | | | [°C] |
| 15% Polydioxanone | Polydioxanone and | Crystalline Polylactide | 54 |
| 85% Poly (L-lactide) | Amorphous Polylactide | | |
| 20% Polydioxanone | Polydioxanone and | Crystalline Polylactide | 45 |
| 80% Poly (L-lactide) | Amorphous Polylactide | | |
| 15% Trimethylene Carbonate | Trimethylene Carbonate and | Crystalline Polylactide | 54 |
| 85% Poly (L-lactide) | Amorphous Polylactide | | |
| 20% Trimethylene Carbonate | Trimethylene Carbonate and | Crystalline Polylactide | 55 |
| 80% Poly (L-lactide) | Amorphous Polylactide | | |

The copolymers in Table 1 may undergo a partial shift when approaching T_{g} and T_{Trans} may be depressed when the materials are in aqueous solution. Since these polymers degrade by water absorption and bulk hydrolysis, water molecules entering the polymer matrices may act as plasticizers, causing the soft segments to soften at lower temperatures than in dry air. Thus, polymers exhibiting T_{Trans} depression in aqueous solution may maintain a temporary shape through temperature excursions in the dry state, such as during shipping and storage, and shape shift to its permanent shape at body temperatures upon implantation.

Thus, in embodiments, the shape memory polymer may include a block copolymer of polydioxanone and polylactide with the polydioxanone present in an amount from about 5 mol% to about 20 mol% of the copolymer, in embodiments from about 15 mol% to about 19 mol% of the copolymer, and the polylactide present in an amount from about 80 mol% to about 95 mol% of the copolymer, in embodiments from about 81 mol% to about 85 mol% of the copolymer. In other embodiments, the shape memory polymer may include a block copolymer of trimethylene carbonate and polylactide, with the trimethylene carbonate present in an amount from about 5 mol% to about 20 mol% of the copolymer, in embodiments from about 15 mol% to about 19 mol% of the copolymer, and the polylactide may be present in an amount from about 80 mol% to about 95 mol% of the copolymer, in embodiments from about 81 mol% to about 85 mol% of the copolymer.

It is envisioned that T_{Trans} may be tailored by changing block segment molar ratios, polymer molecular weight, and time allowed for hard segment formation. In embodiments, T_{Trans} may be tailored by blending various amounts of low molecular weight oligomers of the soft segment domain into the copolymer. Such oligomers may segregate to soft domains and act as plasticizers to cause a downward shift in T_{Trans}.

Additionally, the copolymers forming thread 11 may include emulsifying agents, solubilizing agents, wetting agents, taste modifying agents, plasticizers, active agents, water soluble inert fillers, preservatives, buffering agents, coloring agents, and stabilizers. The addition of a plasticizer to the formulation can improve flexibility. The plasticizer or mixture of plasticizers may be polyethylene glycol, glycerol, sorbitol, sucrose, corn syrup, fructose, dioctyl-sodium sulfosuccinate, triethyl citrate, tributyl citrate, 1,2-propylenglycol, mono-, di- or triacetates of glycerol, or natural gums.

In some embodiments, crystalline degradable salts or minerals may be added to the block copolymer compositions to create polymer composites which may improve shape memory properties. An example of such a composite using polylactide homopolymer and crystalline hydroxyapatite is described in Zheng et al., "Shape memory properties of poly (D,L-lactide/hydroxyapatite composites," Biomaterials, 27 (2006) 4288-4295.

Other shape memory materials, including shape memory metals and metal alloys such as Nitinol, may also be used to form thread 11.

In embodiments, a molding process may be utilized to produce thread 11. Plastic molding methods are within the purview of those skilled in the art and include, but are not limited to, melt molding, solution molding, and the like. Injection molding, extrusion molding, compression molding and other methods can also be used as the melt molding technique. Once placed in the mold with the proper dimensions and configuration, the polymeric material used to form thread 11 may be heated to a suitable temperature, such as the permanent temperature (Tₚₑᵣₘ) which may, in embodiments, be the melting temperature of the shape memory polymeric material utilized to form the surgical suture. Heating of thread 11 may be at suitable temperatures including, for example, from about 40°C to about 180°C, in some embodiments from about 80°C to about 150°C, for a period of time of from about 2 minutes to about 60 minutes, in other embodiments from about 15 minutes to about 20 minutes, to obtain the permanent shape and dimensions.

The temperature for deformation treatment of thread 11 and/or end effector 10 molded with a previously memorized shape is one that makes possible ready deformation without producing cracks and should not exceed the temperature adopted for the shape memorization (e.g., Tₚₑᵣₘ). Deformation treatment at a temperature exceeding that for the original shape memorization may cause the object to memorize/program a new deformed shape. After thread 11 and/or end effector 10 with the desired shape has been formed, thread 11 and/or end effector 10 may be deformed at above Tₜᵣₐₙₛ to obtain an alternate, temporary configuration.

Suitable temperatures for deformation will vary depending on the shape memory polymer utilized, but generally may be above the transition temperature of the polymer (Tₜᵣₐₙₛ), but below the T_{perm.} In embodiments, the shape memory polymer is cooled from its Tₚₑᵣₘ to a lower temperature which remains above the Tₜᵣₐₙₛ and deformed, in embodiments by hand and/or mechanical means. In other embodiments, the surgical suture is deformed at room temperature (about 20° C to about 25° C) to obtain its temporary shape, although the temperature may differ depending upon the particular polymer employed. The surgical suture may then be cooled to a temperature below the Tₜᵣₐₙₛ of the material utilized to form the suture, at which time thread 11 is ready for use. As the Tₜᵣₐₙₛ is usually greater than room temperature, in some embodiments, cooling to room temperature is sufficient to lock in the temporary shape.

There are no particular limitations on the manner in which the deformation can be achieved. Deformation can be achieved either by hand or by means of a suitable device selected to provide the desired temporary configuration to thread 11 and/or end effector 10.

In some embodiments, to keep the shape of thread 11 and/or end effector 10 in its temporary shape, thread 11 should be stored at a temperature which will not cause transition to the permanent shape. In some embodiments, thread 11 may be stored in a refrigerator.

In other embodiments, the shape memory polymeric materials of the present disclosure may be compressed or expanded into temporary forms that are smaller or larger in diameter than their permanent shape. As will be discussed in further detail below, in this manner, loop 12 may be tightened or loosened depending on the desired application.

Thus prepared, thread 11 recovers its permanent shape upon application of energy, such as on heating, either by placement in a patient's body, or the addition of exogenous heat at a prescribed temperature, in certain embodiments above the Tₜᵣₐₙₛ of the shape memory polymer utilized. As thread 11 is utilized in a living body, heating with body heat (about 37° C) is possible. In such a case, the temperature for shape programming should be as low as possible and the recovery of the permanent shape may occur fairly slowly. In embodiments, recovery of the permanent shape may occur from about 1 second to about 5 seconds after insertion into tissue.

However, in some embodiments a higher shape memory temperature may be desirable in order to make the shape recover at a slightly higher temperature than body temperature. Thus, in some cases, releasing thread 11 from deformation to recover the permanent shape can be achieved by heating. On heating at a temperature of from about 30° C to about 50° C, in embodiments from about 39° C to about 43° C, the temporary shape may be released and the permanent shape recovered. The higher the temperature for heating, the shorter the time for recovery of the originally memorized shape. The means for this heating is not limited. Heating can be accomplished by using a gas or liquid heating medium, heating devices, ultrasonic waves, electrical induction, and the like. Of course, in an application involving a living body, care must be taken to utilize a heating temperature which will not cause bums. Examples of liquid heating media include, physiological saline solution, alcohol, combinations thereof, and the like.

Similarly, in other embodiments, electrically active polymers, also known as electroactive polymers, which can alter their configuration upon application of electricity, may be utilized to fashion thread 11. Suitable examples of electroactive polymers include poly(aniline), substituted poly(aniline)s, polycarbazoles, substituted polycarbazoles, polyindoles, poly(pyrrole)s, substituted poly(pyrrole)s, poly(thiophene)s, substituted poly(thiophene)s, poly(acetylene)s, poly(ethylene dioxythiophene)s, poly(ethylenedioxypyrrole)s, poly(p-phenylene vinylene)s, and the like, or combinations including at least one of the foregoing electroactive polymers. Blends or copolymers or composites of the foregoing electroactive polymers may also be used.

Similar to the change in shape which a shape memory material may undergo upon the application of energy, such as heat, in some embodiments, an electroactive polymer may undergo a change in shape upon the application of electricity from a low voltage electrical source (such as a battery). Suitable amounts of electricity which may be applied to effect such change will vary with the electroactive polymer utilized, but can be from about 5 volts to about 30 volts; in other embodiments, from about 10 volts to about 20 volts. The application of electricity will result in thread 11 constructed of the electroactive polymer changing its shape.

While an electroactive polymer does not have the same permanent shape and temporary shape as those terms are described above with respect to shape memory polymers, as used herein the term "permanent shape" as applied to an electroactive polymer means the shape the electroactive polymer adopts upon the application of electricity, and the term "temporary shape" as applied to an electroactive polymer means the shape of the electroactive polymer adopts in the absence of electricity.

Filaments used for forming sutures of the present disclosure may be formed using any technique within the purview of those skilled in the art, such as, for example, extrusion, molding and/or solvent casting.

In embodiments, the suture of the present disclosure may include a yarn made of more than one filament, which may contain multiple filaments of the same or different materials.

As used herein, the terms "fibers", "filaments" and "yarns" each may be used to construct sutures, in whole or in part. The term "fibers," in this context, are generally used to designate natural or synthetic structures that have a length approximately 3 orders of magnitude greater than their diameter or width. The term "filaments" are typically used to describe "fibers" of indefinite or extreme length, and "yarns" as a generic term for a continuous strand of twisted or untwisted "fibers" or "filaments" in a form suitable for knitting, weaving, braiding or otherwise intertwining.

In embodiments, sutures of the present disclosure may possess a core/sheath configuration, fibers may possess a core/sheath configuration, yarns may possess a core/sheath configuration, or both. Any material described herein, including the shape memory materials described above, may be utilized to form the core, the sheath, or both.

Sutures of the present disclosure may be monofilament or multifilament (e.g. braided). Methods for making sutures from these suitable materials are within the purview of those skilled in the art (e.g. extrusion and molding). The filaments may be combined to create a multifilament suture using any technique within the purview of one skilled in the art such as commingling, twisting, braiding, weaving, entangling, and knitting. For example, filaments may be combined to form a yarn or they may be braided. In another example, filaments may be combined to form a yarn and then those multifilament yarns may be braided. Those skilled in the art reading this disclosure will envision other ways in which filaments may be combined. Fibers may also be combined to produce a non-woven multifilament large diameter suture. In certain embodiments, a multifilament structure useful in forming a suture according to the present disclosure may be produced by braiding. The braiding can be done by any method within the purview of those skilled in the art. For example, braid constructions for sutures and other medical devices are described in U.S. Patent Nos. 5,019,093; 5,059,213; 5,133,738; 5,181,923; 5,226,912; 5,261,886; 5,306,289; 5,318,575; 5,370,031; 5,383,387; 5,662,682; 5,667,528; and 6,203,564. Furthermore, the suture may include portions which are monofilament and portions which are multifilament.

Once the suture is constructed, it can be sterilized by any means within the purview of those skilled in the art.

Therapeutic agents may be utilized with thread 11, e.g., coated or impregnated therewith. Therapeutic agents, sometimes referred to herein as bioactive agents, include, but are not limited to, drugs, amino acids, peptides, polypeptides, proteins, polysaccharides, muteins, immunoglobulins, antibodies, cytokines (e.g., lymphokines, monokines, chemokines), blood clotting factors, hemopoietic factors, interleukins (1 through 18), interferons (β-IFN, α-IFN and γ-IFN), erythropoietin, nucleases, tumor necrosis factor, colony stimulating factors (e.g., GCSF, GM-CSF, MCSF), insulin, anti-tumor agents and tumor suppressors, blood proteins, fibrin, thrombin, fibrinogen, synthetic thrombin, synthetic fibrin, synthetic fibrinogen, gonadotropins (e.g., FSH, LH, CG, etc.), hormones and hormone analogs (e.g., growth hormone, luteinizing hormone releasing factor ), vaccines (e.g., tumoral, bacterial and viral antigens); somatostatin; antigens; blood coagulation factors; growth factors (e.g., nerve growth factor, insulin-like growth factor); bone morphogenic proteins, TGF-B, protein inhibitors, protein antagonists, and protein agonists; nucleic acids, such as antisense molecules, DNA, RNA, RNAi; oligonucleotides; polynucleotides; cells, viruses, and ribozymes.

In some embodiments, the therapeutic agent includes at least one of the following drugs, including combinations and alternative forms of the drugs such as alternative salt forms, free acid form, free base forms, pro-drugs and hydrates: analgesics/antipyretics (e.g., aspirin, acetaminophen, ibuprofen, naproxen sodium, buprenorphine, propoxyphene hydrochloride, propoxyphene napsylate, meperidine hydrochloride, hydromorphone hydrochloide, morphine, oxycodone, codeine, dihydrocodeine bitartrate, pentazocine, hydrocodone bitartrate, levorphanol, diflunisal, trolamine salicylate, nalbuphine hydrochloride, mefenamic acid, butorphanol, choline salicylate, butalbital, phenyltoloxamine citrate, diphenhydramine citrate, methotrimeprazine, cinnamedrine hydrochloride, and meprobamate); antiasthmatics (e.g., ketotifen and traxanox); antibiotics (e.g., neomycin, streptomycin, chloramphenicol, cephalosporin, ampicillin, penicillin, tetracycline, and ciprofloxacin); antidepressants (e.g., nefopam, oxypertine, doxepin, amoxapine, trazodone, amitriptyline, maprotiline, phenelzine, desipramine, nortriptyline, tranylcypromine, fluoxetine, doxepin, imipramine, imipramine pamoate, isocarboxazid, trimipramine, and protriptyline); antidiabetics (e.g., biguanides and sulfonylurea derivatives); antifungal agents (e.g., griseofulvin, ketoconazole, itraconizole, amphotericin B, nystatin, and candicidin); antihypertensive agents (e.g., propanolol, propafenone, oxyprenolol, nifedipine, reserpine, trimethaphan, phenoxybenzamine, pargyline hydrochloride, deserpidine, diazoxide, guanethidine monosulfate, minoxidil, rescinnamine, sodium nitroprusside, rauwolfia serpentina, alseroxylon, and phentolamine); anti-inflammatories (e.g., (non-steroidal) indomethacin, ketoprofen, flurbiprofen, naproxen, ibuprofen, ramifenazone, piroxicam, (steroidal) cortisone, dexamethasone, fluazacort, celecoxib, rofecoxib, hydrocortisone, prednisolone, and prednisone); antineoplastics (e.g., cyclophosphamide, actinomycin, bleomycin, dactinomycin, daunorubicin, doxorubicin, epirubicin, mitomycin, methotrexate, fluorouracil, gemcitabine, carboplatin, carmustine (BCNU), methyl-CCNU, cisplatin, etoposide, camptothecin and derivatives thereof, phenesterine, paclitaxel and derivatives thereof, docetaxel and derivatives thereof, vinblastine, vincristine, goserelin, leuprolide, tamoxifen, interferon alfa, retinoic acid (ATRA), nitrogen mustard alkylating agents, and piposulfan); antianxiety agents (e.g., lorazepam, buspirone, prazepam, chlordiazepoxide, oxazepam, clorazepate dipotassium, diazepam, hydroxyzine pamoate, hydroxyzine hydrochloride, alprazolam, droperidol, halazepam, chlormezanone, and dantrolene); immunosuppressive agents (e.g., cyclosporine, azathioprine, mizoribine, and FK506 (tacrolimus)); antimigraine agents (e.g., ergotamine, propanolol, isometheptene mucate, and dichloralphenazone); sedatives/hypnotics (e.g., barbiturates such as pentobarbital, pentobarbital, and secobarbital; and benzodiazapines such as flurazepam hydrochloride, triazolam, and midazolam); antianginal agents (e.g., beta-adrenergic blockers; calcium channel blockers such as nifedipine, and diltiazem; and nitrates such as nitroglycerin, isosorbide dinitrate, pentearythritol tetranitrate, and erythrityl tetranitrate); antipsychotic agents (e.g., haloperidol, loxapine succinate, loxapine hydrochloride, thioridazine, thioridazine hydrochloride, thiothixene, fluphenazine, fluphenazine decanoate, fluphenazine enanthate, trifluoperazine, chlorpromazine, perphenazine, lithium citrate, and prochlorperazine); antimanic agents (e.g., lithium carbonate); antiarrhythmics (e.g., bretylium tosylate, esmolol, verapamil, amiodarone, encainide, digoxin, digitoxin, mexiletine, disopyramide phosphate, procainamide, quinidine sulfate, quinidine gluconate, quinidine polygalacturonate, flecainide acetate, tocainide, and lidocaine); antiarthritic agents (e.g., phenylbutazone, sulindac, penicillanine, salsalate, piroxicam, azathioprine, indomethacin, meclofenamate, gold sodium thiomalate, ketoprofen, auranofin, aurothioglucose, and tolmetin sodium); antigout agents (e.g., colchicine, and allopurinol); anticoagulants (e.g., heparin, heparin sodium, and warfarin sodium); thrombolytic agents (e.g., urokinase, streptokinase, and alteplase); antifibrinolytic agents (e.g., aminocaproic acid); hemorheologic agents (e.g., pentoxifylline); antiplatelet agents (e.g., aspirin); anticonvulsants (e.g., valproic acid, divalproex sodium, phenytoin, phenytoin sodium, clonazepam, primidone, phenobarbitol, carbamazepine, amobarbital sodium, methsuximide, metharbital, mephobarbital, mephenytoin, phensuximide, paramethadione, ethotoin, phenacemide, secobarbitol sodium, clorazepate dipotassium, and trimethadione); antiparkinson agents (e.g., ethosuximide); antihistamines/antipruritics (e.g., hydroxyzine, diphenhydramine, chlorpheniramine, brompheniramine maleate, cyproheptadine hydrochloride, terfenadine, clemastine fumarate, triprolidine, carbinoxamine, diphenylpyraline, phenindamine, azatadine, tripelennamine, dexchlorpheniramine maleate, methdilazine, and); agents useful for calcium regulation (e.g., calcitonin, and parathyroid hormone); antibacterial agents (e.g., amikacin sulfate, aztreonam, chloramphenicol, chloramphenicol palirtate, ciprofloxacin, clindamycin, clindamycin palmitate, clindamycin phosphate, metronidazole, metronidazole hydrochloride, gentamicin sulfate, lincomycin hydrochloride, tobramycin sulfate, vancomycin hydrochloride, polymyxin B sulfate, colistimethate sodium, and colistin sulfate); antiviral agents (e.g., interferon alpha, beta or gamma, zidovudine, amantadine hydrochloride, ribavirin, and acyclovir); antimicrobials (e.g., cephalosporins such as cefazolin sodium, cephradine, cefaclor, cephapirin sodium, ceftizoxime sodium, cefoperazone sodium, cefotetan disodium, cefuroxime e azotil, cefotaxime sodium, cefadroxil monohydrate, cephalexin, cephalothin sodium, cephalexin hydrochloride monohydrate, cefamandole nafate, cefoxitin sodium, cefonicid sodium, ceforanide, ceftriaxone sodium, ceftazidime, cefadroxil, cephradine, and cefuroxime sodium; penicillins such as ampicillin, amoxicillin, penicillin G benzathine, cyclacillin, ampicillin sodium, penicillin G potassium, penicillin V potassium, piperacillin sodium, oxacillin sodium, bacampicillin hydrochloride, cloxacillin sodium, ticarcillin disodium, azlocillin sodium, carbenicillin indanyl sodium, penicillin G procaine, methicillin sodium, and nafcillin sodium; erythromycins such as erythromycin ethylsuccinate, erythromycin, erythromycin estolate, erythromycin lactobionate, erythromycin stearate, and erythromycin ethylsuccinate; and tetracyclines such as tetracycline hydrochloride, doxycycline hyclate, and minocycline hydrochloride, azithromycin, clarithromycin); anti-infectives (e.g., GM-CSF); bronchodilators (e.g., sympathomimetics such as epinephrine hydrochloride, metaproterenol sulfate, terbutaline sulfate, isoetharine, isoetharine mesylate, isoetharine hydrochloride, albuterol sulfate, albuterol, bitolterolmesylate, isoproterenol hydrochloride, terbutaline sulfate, epinephrine bitartrate, metaproterenol sulfate, epinephrine, and epinephrine bitartrate; anticholinergic agents such as ipratropium bromide; xanthines such as aminophylline, dyphylline, metaproterenol sulfate, and aminophylline; mast cell stabilizers such as cromolyn sodium; inhalant corticosteroids such as beclomethasone dipropionate (BDP), and beclomethasone dipropionate monohydrate; salbutamol; ipratropium bromide; budesonide; ketotifen; salmeterol; xinafoate; terbutaline sulfate; triamcinolone; theophylline; nedocromil sodium; metaproterenol sulfate; albuterol; flunisolide; fluticasone proprionate; steroidal compounds and hormones (e.g., androgens such as danazol, testosterone cypionate, fluoxymesterone, ethyltestosterone, testosterone enathate, methyltestosterone, fluoxymesterone, and testosterone cypionate; estrogens such as estradiol, estropipate, and conjugated estrogens; progestins such as methoxyprogesterone acetate, and norethindrone acetate; corticosteroids such as triamcinolone, betamethasone, betamethasone sodium phosphate, dexamethasone, dexamethasone sodium phosphate, dexamethasone acetate, prednisone, methylprednisolone acetate suspension, triamcinolone acetonide, methylprednisolone, prednisolone sodium phosphate, methylprednisolone sodium succinate, hydrocortisone sodium succinate, triamcinolone hexacetonide, hydrocortisone, hydrocortisone cypionate, prednisolone, fludrocortisone acetate, paramethasone acetate, prednisolone tebutate, prednisolone acetate, prednisolone sodium phosphate, and hydrocortisone sodium succinate; and thyroid hormones such as levothyroxine sodium); hypoglycemic agents (e.g., human insulin, purified beef insulin, purified pork insulin, glyburide, chlorpropamide, glipizide, tolbutarnide, and tolazamide); hypolipidemic agents (e.g., clofibrate, dextrothyroxine sodium, probucol, pravastitin, atorvastatin, lovastatin, and niacin); proteins (e.g., DNase, alginase, superoxide dismutase, and lipase); nucleic acids (e.g., sense or anti-sense nucleic acids encoding any therapeutically useful protein, including any of the proteins described herein); agents useful for erythropoiesis stimulation (e.g., erythropoietin); antiulcer/antireflux agents (e.g., famotidine, cimetidine, and ranitidine hydrochloride); antinauseants/antiemetics (e.g., meclizine hydrochloride, nabilone, prochlorperazine, dimenhydrinate, promethazine hydrochloride, thiethylperazine, and scopolamine); as well as other drugs useful in the compositions and methods described herein include mitotane, halonitrosoureas, anthrocyclines, ellipticine, ceftriaxone, ketoconazole, ceftazidime, oxaprozin, albuterol, valacyclovir, urofollitropin, famciclovir, flutamide, enalapril, mefformin, itraconazole, buspirone, gabapentin, fosinopril, tramadol, acarbose, lorazepan, follitropin, glipizide, omeprazole, fluoxetine, lisinopril, tramsdol, levofloxacin, zafirlukast, interferon, growth hormone, interleukin, erythropoietin, granulocyte stimulating factor, nizatidine, bupropion, perindopril, erbumine, adenosine, alendronate, alprostadil, benazepril, betaxolol, bleomycin sulfate, dexfenfluramine, diltiazem, fentanyl, flecainid, gemcitabine, glatiramer acetate, granisetron, lamivudine, mangafodipir trisodium, mesalamine, metoprolol fumarate, metronidazole, miglitol, moexipril, monteleukast, octreotide acetate, olopatadine, paricalcitol, somatropin, sumatriptan succinate, tacrine, verapamil, nabumetone, trovafloxacin, dolasetron, zidovudine, finasteride, tobramycin, isradipine, tolcapone, enoxaparin, fluconazole, lansoprazole, terbinafine, pamidronate, didanosine, diclofenac, cisapride, venlafaxine, troglitazone, fluvastatin, losartan, imiglucerase, donepezil, olanzapine, valsartan, fexofenadine, calcitonin, and ipratropium bromide. In some embodiments, the drug may be water soluble. In other embodiments, the drug may not be water soluble.

Thread 11 may be formed using any technique within the purview of those skilled in the art, such as, for example, extrusion, molding and/or spinning. In some embodiments, thread 11 may include a yarn made of more than one filament, which may contain multiple filaments of the same or different materials. Where thread 11 is made of multiple filaments, thread 11 may be made using any known technique such as, for example, braiding, weaving or knitting. Threads 11 may also be combined to produce a non-woven suture. Threads 11 may be drawn, oriented, crinkled, twisted, commingled or air entangled to form yarns as part of the suture forming process. In one embodiment, a multifilament suture may be produced by braiding. The braiding may be done by any method within the purview of those skilled in the art.

Still referring to FIG. 1, looped suture 10 includes a loop 12 formed on a distal end 10b thereof. Proximal end 10a of looped suture 10 may include one or more suture needles (not shown). Loop 12 forms a substantially teardrop shape and may be of any size. In one embodiment, loop 12 is sized to receive proximal end 10a of looped suture 10. A first section 13 of monofilament thread 11 overlays a second section 14 of thread 11 to form loop 12. The adjacent surfaces of first and second sections 13, 14 form a joined segment or joint 15.

In one embodiment, first and second sections 13, 14 of thread 11 are welded together. In this manner, first and second sections 13, 14 of thread 11 are locally heated until each fuses to form weld segment 15. Various types of energy may be used to locally heat first and second sections 13, 14 to form joined segment 15, including, RF, ultrasonic, laser, electrical arc discharge, and thermal. Alternatively, first and second sections 13, 14 of thread 11 may be joined using glue, epoxy or other adhesive.

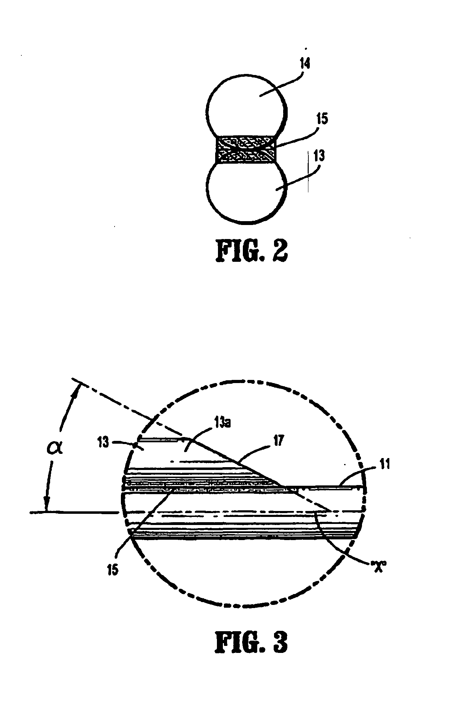

With particular reference to FIG. 3, a proximal end 13a of first section 13 is angled to form a tapered surface 17. Tapered surface 17 angles downwardly towards proximal end 10a of looped suture 10. Tapered surface 17 forms an angle α relative to a longitudinal axis "X" of second section 14, between zero degrees (0°) and ninety degrees (90°), and preferably between about five degrees (5°) to about sixty degrees (60°). Tapered surface 17 facilitates insertion of loop 12 into or through tissue. Tapered surface 17 may be formed prior to, during or following the joining of first and second sections 13, 14. In one embodiment, tapered surface 17 is formed during the welding process using a die (not shown) having a cutting surface (not shown). In another embodiment, tapered surface 17 is formed by a blade (not shown). The blade used to form tapered surface 17 may be heated, ultrasonically vibrated or otherwise adapted to facilitate cutting of thread 11. Tapered surface 17 of first section 13 may be formed such that joined segment 15 extends beyond first section 13 of thread 11. In this manner, tapered surface 17 forms a smooth transition with second section 14 of thread 11, thereby decreasing the likelihood that first and second sections 13, 14 might separate or peel away from each other as looped suture 10 is pulled through tissue.

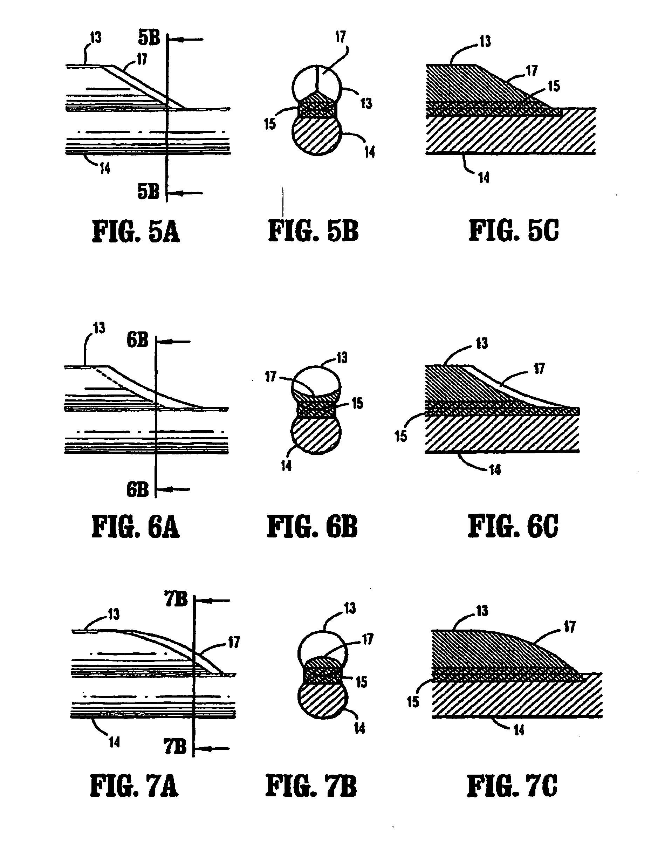

Although shown having a substantially planar taper, tapered surface 17 may include any number of configurations. For example, FIGS. 5A-7C illustrate alternate embodiments, including a beveled tapered surface 17a (FIGS. 5A-5C), a laterally and longitudinally concave tapered surface 17b (FIGS. 6A-6C), a laterally and longitudinally convex tapered surface 17c (FIGS. 7A-7C) or any combination thereof. Respective beveled, concave and convex tapered surfaces (collectively, contoured tapered surfaces 17a-c) may be formed in a similar manner as planar tapered surface 17. That is, contoured tapered surfaces 17a-c may be formed during the welding process using a die (not shown) having an appropriately shaped cutting surface (not shown). Alternatively, contoured tapered surfaces 17a-c may be formed using a blade (not shown) having an appropriately shaped cutting surface. Tapered surface 17 may be selected depending on the tissue being sutured and/or the desired depth of penetration of loop 12 within the tissue.

Turning briefly to FIG. 1A, looped suture 10 may include barbs 3, or hooks, latches protrusions, leaves, teeth, other projections or combinations thereof (not shown), formed therein and/or thereon. Barbs 3 may be arranged in any suitable pattern, for example, helical, linear, or randomly spaced. The pattern may be symmetrical or asymmetrical. The number, configuration, spacing and surface area of barbs 3 may vary depending upon the tissue in which suture 10 is used, as well as the composition and geometry of the material of thread 11. Additionally, the proportions of barbs 3 may remain relatively constant while the overall length of barbs 3 and the spacing of barbs 3 may be determined by the tissue being connected. For example, if suture 10 is to be used to connect the edges of a wound in skin or tendon, barbs 3 may be made relatively short and more rigid to facilitate entry into this rather firm tissue. Alternatively, if suture 10 is intended for use in fatty tissue, which is relatively soft, barbs 3 may be made longer and spaced further apart to increase the ability of suture 10 to grip the soft tissue.

The surface area of barbs 3 may also vary. For example, fuller-tipped barbs may be made of varying sizes designed for specific surgical applications. For joining fat and relatively soft tissues, larger barbs may be desired, whereas smaller barbs may be more suitable for collagen-dense tissues. In some embodiments, a combination of large and small barbs within the same structure may be beneficial, for example when a suture is used in tissue repair with differing layer structures. Use of the combination of large and small barbs with the same suture wherein barb sizes are customized for each tissue layer will ensure maximum anchoring properties. In particular embodiments, a single directional suture may have both large and small barbs; in other embodiments a bi-directional suture may have both large and small barbs. Barbs 3 may include geometrical shapes such as round, triangular, square, oblique, elliptical, octagonal, rectangular, and flat. In some embodiments, barbs 3 may be formed on loop 12 which allows movement of loop 12 through tissue in one direction but resists the withdrawal of suture 10 after loop 12 has been implanted in the tissue.

When fabricated from a degradable material, suture 10 maintains its structural integrity after implantation for a predetermined period of time, depending on the characteristics of the particular copolymer used. Such characteristics include, for example, the components of the copolymer, including both the monomers utilized to form the copolymer and any additives thereto, as well as the processing conditions (e.g., rate of copolymerization reaction, temperature for reaction, pressure, etc.), and any further treatment of the resulting copolymers, i.e., coating, sterilization, etc. The manufacturing parameters involved in the forming of loop 12 also affect the rate at which suture 10 is absorbed. Joint 15 may absorb at a different rate from the remainder of suture 10.

The formation of barbs 3 (FIG. 1A) on a suture body may be utilized to change the degradation time of suture 10 as described in U.S. Patent Application No. 11/556,002 filed on November 2, 2006, entitled "Long Term Bioabsorbable Barbed Sutures". U.S. Patent No. 8, 353, 931.

Therapeutic agents described above may be applied onto suture 10 utilizing any method within the purview of one skilled in the art including, for example, dipping, spraying, vapor deposition, brushing, solvent evaporation, compounding and the like. In embodiments, a bioactive agent may be deposited within the barb angles, that is, the angle formed between barbs 3 and thread 11. This placement of the bioactive agent between barb 3 and thread 11 places the bioactive agent at precisely defined locations within a tissue wound closure, which thereby provides a unique controlled and sustained release dosage form.

Thread 11 may be dyed in order to increase the visibility of suture 10 in the surgical field. Any dye suitable for incorporation in medical devices may be used. Such dyes include, but are not limited to, carbon black, bone black, D&C Green No. 6, and D&C Violet No. 2. Filaments in accordance with the present disclosure may be dyed by adding dye in an amount up to about a few percent; in other embodiments, they may be dyed by adding dye in an amount of about 0.2%; in still further embodiments, the dye may be added in an amount from about 0.06% to about 0.08%.

In use, looped suture 10 includes a needle (not shown) on proximal end 10a thereof. The needle is inserted into and through a first and second flap of tissue. Looped suture 10 is pulled through the tissue until proximal end 13a of first section 13 contacts the tissue. Continued pulling on proximal end 10a of suture 10 causes tapered proximal end 13a to engage the tissue. Tapered surface 17 of proximal end 13a allows the overlapping section of suture 10 to be received within the tissue with reduced resistance while minimizing trauma to the tissue. Once a portion of loop 12 of suture 10 is received within the tissue, proximal end 10a of suture 10 may be inserted through loop 12. Proximal end 10a of suture 10 may then be pulled tight, thereby approximating the first and second tissue flaps towards one another. Proximal end 10a of suture 10 may then be knotted or otherwise secured to loop 12. In one embodiment, a knot may be formed in proximal end 10a to prevent proximal end 10a from withdrawing from loop 12. In another embodiment, proximal end 10a of suture 10 may be tied directly to loop 12.

As discussed above, the thread forming the looped sutures of the present disclosure may be formed entirely, or in part, of a shape memory material. Such materials include a first or permanent configuration, and a second or temporary configuration. Transformation from the temporary configuration to the permanent configuration may result in radial expansion or contraction, axial lengthening or shortening, and/or reorientation of the thread along a length thereof.

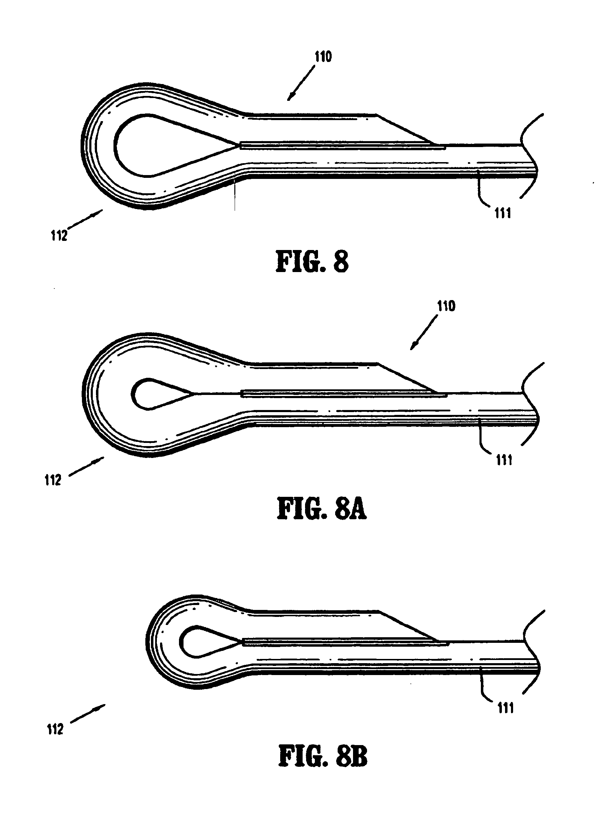

With reference now to FIGS. 8-8B, in some embodiments, looped suture 110 is configured such that at least a portion of loop 112 is composed of a shape memory polymeric material. For example, a first side of thread 111 corresponding to the inner portion of loop 112 may be at least partially composed of shape memory polymeric material(s). When the shape memory polymeric material of loop 112 is configured to radial expand (FIG. 8A) and/or axially shorten (FIG. 8B) along a length thereof, the opening formed by loop 112 will contract or shrink during the transition from the temporary configuration to the permanent configuration. In this manner, the contracting of the opening formed by loop 112 may capture or tighten about proximal end of looped suture 110 received therethrough.

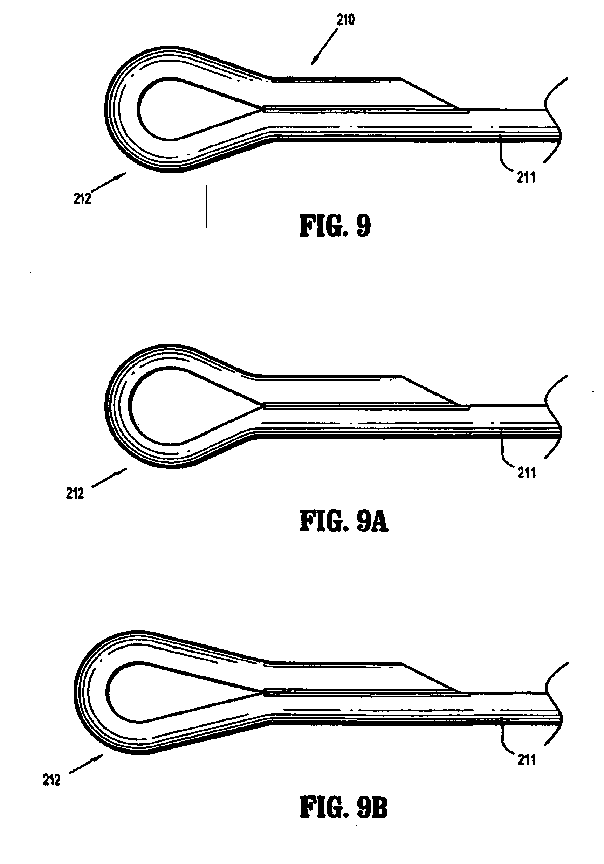

Turing now to FIGS. 9-9B, in an alternate embodiment, thread 211 is configured such that the shape memory polymeric material of loop 212 is configured to radial contract (FIG. 9A) and/or axially lengthen (FIG. 9B) along a length thereof. As such, the opening formed in loop 212 expands or loosen during the transition from the temporary configuration to the permanent configuration. In this manner, a proximal end of suture 210 may be released from within the opening formed by loop 212. In other embodiments, loosening of the opening formed in loop 12 causes the release of a therapeutic agent therefrom.

Alternatively, or in addition, the looped sutures according to the present disclosure are configured to include portions of shape memory polymeric material positioned about the loop such that loop changes configurations. The loop may assume a flattened or tear drop shape when in a temporary configuration, and upon transitioning to the permanent configuration, the loop becomes more bulbous. In this manner, when the shape memory portions of the loop are in the temporary configuration, the looped suture can be more easily received through tissue, and upon transitioning, the looped suture becomes more secure engaged with the tissue. Alternatively, the looped suture is configured such that the loop assumes a substantially bulbous shape in the temporary configuration, and upon transitioning, the loop becomes narrower. In this manner, the looped suture is configured to be more easily pulled through tissue once the loop has transitioned from the temporary configuration to the permanent configuration.

Although the illustrative embodiments of the present disclosure have been described herein with reference to the accompanying drawings, it is to be understood that the disclosure is not limited to those precise embodiments, and that various other changes and modifications may be effected therein by one skilled in the art without departing from the scope of the disclosure.

## Claims

1. A suture (10; 110) comprising:
an elongate body (11; 111) including a proximal end and a distal end;
a loop (12; 112) integrally formed on the distal end of the elongate body (11; 111), wherein at least a portion of the loop (12; 112) includes a shape memory polymeric material; and
wherein the shape memory polymeric material is configured to radially expand and axially shorten when transitioning from a temporary configuration to a permanent configuration, and the radial expansion and axial shortening causes constricting of the loop (12; 112).

2. The suture (10; 110) of claim 1, wherein the loop (12; 112) is substantially bulbous when the shape memory polymeric material is in a temporary configuration.

3. The suture (10; 110) of claim 2, wherein the loop (12; 112) is substantially flattened when the shape memory polymeric material transitions from the temporary configuration to the permanent configuration.

4. The suture (10; 110) of any preceding claim, wherein the shape memory polymeric material is configured such that the loop (12; 112) flattens during the transition from a temporary configuration to a permanent configuration.

5. The suture (10; 110) of any preceding claim, wherein the shape memory polymer is selected from the group consisting of degradable materials, non-degradable materials, and combinations thereof.

6. The suture (10; 110) of claim 5, wherein the shape memory polymer comprises a non-degradable material selected from the group consisting of polyolefins, polyethylene glycols, polyethylene oxides, polyolefin copolymers, fluorinated polyolefins, polyamides, polyamines, polyimines, polyesters, polyethers, polybutesters, polyurethanes, acrylic polymers, methacrylics polymers, vinyl halide polymers and copolymers, polyvinyl alcohols, polyvinyl ethers, polyvinylidene halides, polychlorofluoroethylene, polyacrylonitrile, polyaryletherketones, polyvinyl ketones, polyvinyl aromatics, polyvinyl esters, copolymers of vinyl monomers, acrylonitrile-styrene copolymers, ABS resins, ethylene-vinyl acetate copolymers, alkyd resins, polycarbonates, polyoxymethylenes, polyphosphazines, polyimides, epoxy resins, aramids, rayons, spandex, silicones, and combinations thereof.

7. The suture (10; 110) of claim 5, wherein the shape memory polymer comprises a bioabsorbable material selected from the group consisting of aliphatic polyesters, polyamides, polyamines, polyalkylene oxalates, poly(anhydrides), polyamidoesters, copoly(ether-esters), poly(carbonates), poly(hydroxyalkanoates), polyimide carbonates, poly(imino carbonates), polyorthoesters, polyoxaesters, polyphosphazenes, poly(propylene fumarates), polyurethanes, polymer drugs, biologically modified bioabsorbable polymers, and copolymers, homopolymers, and combinations thereof.

8. The suture (10; 110) of claim 5, wherein the shape memory polymer comprises an aliphatic polyester selected from the group consisting of homopolymers and copolymers of lactide, glycolide, epsilon-caprolactone, p-dioxanone, trimethylene carbonate, alkyl derivatives of trimethylene carbonate, Δ-valerolactone, β-butyrolactone, γ-butyrolactone, ε-decalactone, hydroxybutyrate, hydroxyvalerate, 1,4-dioxepan-2-one, 1,5-dioxepan-2-one, 6.6-dimethyl-1,4-dioxan-2-one, 2,5-diketomorpholine, pivalolactone, α,α diethylpropiolactone, ethylene carbonate, ethylene oxalate, 3-methyl-1,4-dioxane-2,5-dione, 3,3-diethyl-1,4-dioxan-2,5-dione, 6,8-dioxabicycloctane-7-one, and combinations thereof.

9. The suture (10; 110) of claim 5, wherein the shape memory polymer comprises a biodegradable polymer selected from the group consisting of poly(amino acids), collagen, elastin, fibrin, fibrinogen, silk, albumin, peptides including sequences for laminin and fibronectin, hyaluronic acid, dextran, alginate, chitin, chitosan, cellulose, glycosaminoglycan, gut, methyl cellulose, ethyl cellulose, hydroxypropyl cellulose, hydroxypropyl methyl cellulose, hydroxybutyl methyl cellulose, cellulose acetate, cellulose propionate, cellulose acetate butyrate, cellulose acetate phthalate, carboxymethyl cellulose, cellulose triacetate, cellulose sulphate sodium salt, nitrocelluloses, chitosan, and combinations thereof.

10. The suture (10; 110) of claim 5, wherein the shape memory polymer comprises a polymer selected from the group consisting of oligo (epsilon-caprolactone) dimethacrylates, oligo (epsilon-caprolactone) butyl acrylates, (n-butyl acrylate), oligo (epsilon caprolactone) diol/oligo (p-dioxanone) diol copolymers, polycaprolactone dimethacrylate poly(butyl acrylate) blends, and combinations thereof.

11. The suture (10; 110) of claim 5, wherein the shape memory polymer comprises a block copolymer of polydioxanone and polylactide; preferably wherein the polydioxanone is present in an amount from about 5 mol% to about 20 mol% of the copolymer and the polylactide is present in an amount from about 80 mol% to about 95 mol% of the copolymer.

12. The suture (10; 110) of claim 5, wherein the shape memory polymer comprises a block copolymer of trimethylene carbonate and polylactide; preferably wherein the trimethylene carbonate is present in an amount from about 5 mol% to about 20 mol% of the copolymer and the polylactide is present in an amount from about 80 mol% to about 85 mol% of the copolymer.

13. The suture (10) of any preceding claim, further comprising a plurality of surface features (3) selected from the group consisting of barbs, hooks, latches, protrusions, leaves, teeth, and combinations thereof; preferably wherein the surface feature (3) comprises a shape memory polymer.

## Patentansprüche

1. Naht (10; 110), Folgendes umfassend:
einen länglichen Körper (11; 111) mit einem proximalen und einem distalen Ende;
eine Schlaufe (12; 112), in einem Stück ausgeformt am länglichen Körper (11; 111), wobei wenigstens ein Teil der Schlaufe (12; 112) ein Formgedächtnispolymermaterial umfasst; und
wobei das Formgedächtnispolymermaterial konfiguriert ist, radial zu expandieren und sich axial zu verkürzen, wenn es von einer temporären Konfiguration in eine permanente Konfiguration übergeht, und das radiale Expandieren und das axiale Verkürzen ein Zusammenziehen der Schlaufe (12; 112) bewirken.

2. Naht (10; 110) nach Anspruch 1, wobei die Schlaufe (12; 112) im Wesentlichen bauchig ist, wenn sich das Formgedächtnispolymermaterial in einer temporären Konfiguration befindet.

3. Naht (10; 110) nach Anspruch 2, wobei die Schlaufe (12; 112) im Wesentlichen abgeflacht wird, wenn das Formgedächtnispolymermaterial von der temporären Konfiguration in die permanente Konfiguration übergeht.

4. Naht (10; 110) nach einem der vorhergehenden Ansprüche, wobei das Formgedächtnispolymermaterial derart konfiguriert ist, dass die Schlaufe (12; 112) beim Übergehen von einer temporären Konfiguration in eine permanente Konfiguration abgeflacht wird.

5. Naht (10; 110) nach einem der vorhergehenden Ansprüche, wobei das Formgedächtnispolymer ausgewählt ist aus der Gruppe bestehend aus zersetzbaren Materialien, beständigen Materialien, nicht beständigen Materialien und Kombinationen aus diesen.

6. Naht (10; 110) nach Anspruch 5, wobei das Formgedächtnispolymer ein beständiges Material umfasst, ausgewählt aus der Gruppe bestehend aus Polyolefinen, Polyethylenglycolen, Polyethylenoxiden, Polyolefincopolymeren, fluorinierten Polyolefinen, Polyamiden, Polyaminen, Polyiminen, Polyestern, Polyethern, Polybutestern, Polyurethanen, Acrylpolymeren, Methacrylpolymeren, Vinylhalidpolymeren und -copolymeren, Polyvinylalkoholen, Polyvinylethern, Polyvinylidenhaliden, Polychlorofluoroethylen, Polyacrylonitril, Polyaryletherketonen, Polyvinylketonen, Polyvinylaromaten, Polyvinylestern, Copolymeren von Vinylmonomeren, Acrylonitrilstyrencopolymeren, ABS-Harzen, Ehylenvinylacetatcopolymeren, Alkydharzen, Polycarbonaten, Polyoxymethylenen, Polyphosphazinen, Polyimiden, Epoxidharzen, Aramiden, Rayon, Spandex, Silikonen und Kombinationen aus diesen.

7. Naht (10; 110) nach Anspruch 5, wobei das Formgedächtnispolymer ein bioabsorbierbares Material umfasst, ausgewählt aus der Gruppe bestehend aus aliphatischen Polyestern, Polyamiden, Polyaminen, Polyalkylenoxalaten, Poly(anhydriden), Polyamidoestern, Copoly(etherestern), Poly(carbonaten), Poly(hydroxyalkanoaten), Polyimidecarbonaten, Poly(iminocarbonaten), Polyorthoestern, Polyoxaestern, Polyphosphazenen, Poly(propylenfumaraten), Polyurethanen, Polymerarzneimitteln, biologisch modifizierten bioabsorbierbaren Polymeren und Copolymeren, Homopolymeren und Kombinationen aus diesen.

8. Naht (10; 110) nach Anspruch 5, wobei das Formgedächtnispolymer einen aliphatischen Polyester umfasst, ausgewählt aus der Gruppe bestehend aus Homopolymeren und Copolymeren von Lactid, Glycolid, Epsiloncaprolacton, p-Dioxanon, Trimethylencarbonat, Alkylderivaten von Trimethylencarbonat, Δ-Valerolacton, β-Butyrolacton, γ-Butyrolacton, ε-Decalacton, Hydroxybutyrat, Hydroxyvalerat, 1,4-Dioxepan-2-on, 1,5-Dioxepan-2-on, 6,6-Dimethyl-1,4-dioxan-2-on, 2,5-Diketomorpholin, Pivalolacton, α,α-Diethylpropiolacton, Ethylenecarbonat, Ethyleneoxalat, 3-Methyl-1,4-dioxane-2,5-dion, 3,3-Diethyl-1,4-dioxan-2,5-dion, 6,8-Dioxabicycloctane-7-on und Kombinationen aus diesen.

9. Naht (10; 110) nach Anspruch 5, wobei das Formgedächtnispolymer ein zersetzbares Polymer umfasst, ausgewählt aus der Gruppe bestehend aus Poly(aminsäuren), Collagen, Elastin, Fibrin, Fibrinogen, Seide, Albumin, Peptiden, einschließlich Sequenzen für Laminin und Fibronectin, Hyaluronsäure, Dextran, Alginat, Chitin, Chitosan, Cellulose, Glycosaminoglycan, Gedärm, Methylcellulose, Ethylcellulose, Hydroxypropylcellulose, Hydroxypropylmethylcellulose, Hydroxybutylmethylcellulose, Celluloseacetat, Cellulosepropionat, Celluloseacetatbutyrat, Celluloseacetatphthalat, Carboxymethylcellulose, Cellulosetriacetat, Cellulosesulphatnatriumsalz, Nitrocellulosen, Chitosan und Kombinationen aus diesen.

10. Naht (10; 110) nach Anspruch 5, wobei das Formgedächtnispolymer ein Polymer umfasst, ausgewählt aus der Gruppe bestehend aus Oligo(epsiloncaprolacton)dimethacrylaten, Oligo(epsiloncaprolacton)butylacrylaten, (n-Butylacrylat), Oligo(epsiloncaprolacton)diol-/Oligo(p-dioxanone)diol-Copolymeren, Polycaprolactondimethacrylat-Poly(butylacrylat)-Mischungen und Kombinationen aus diesen.

11. Naht (10; 110) nach Anspruch 5, wobei das Formgedächtnispolymer ein Block-Copolymer aus Polydioxanon und Polylactid umfasst; wobei vorzugsweise das Polydioxanon in einer Menge von etwa 5 mol% bis etwa 20 mol% des Copolymers vorliegt und das Polylactid in einer Menge von etwa 80 mol% bis etwa 95 mol% des Copolymers vorliegt.

12. Naht (10; 110) nach Anspruch 5, wobei das Formgedächtnispolymer ein Block-Copolymer aus Trimethylencarbonat und Polylactid umfasst; wobei vorzugsweise das Trimethylencarbonat in einer Menge von etwa 5 mol% bis etwa 20 mol% des Copolymers vorliegt und das Polylactid in einer Menge von etwa 80 mol% bis etwa 85 mol% des Copolymers vorliegt.

13. Naht (10) nach einem der vorhergehenden Ansprüche, ferner umfassend mehrere Oberflächenmerkmale (3), ausgewählt aus der Gruppe bestehend aus Widerhaken, Haken, Riegeln, Vorsprüngen, Blättern, Zähnen und Kombinationen aus diesen; wobei das Oberflächenmerkmal (3) vorzugsweise ein Formgedächtnispolymer umfasst.

## Revendications

1. Suture (10 ; 110), comprenant :
un corps allongé (11 ; 111) comprenant une extrémité proximale et une extrémité distale ;
une boucle (12 ; 112) formée d'un seul tenant sur l'extrémité distale du corps allongé (11 ; 111), dans laquelle au moins une portion de la boucle (12 ; 112) comprend un matériau polymère à mémoire de forme ; et
dans laquelle le matériau polymère à mémoire de forme est configuré pour se dilater radialement et se réduire axialement lors de la transition d'une configuration temporaire à une configuration permanente et la dilatation radiale et la réduction axiale provoquent le rétrécissement de la boucle (12 ; 112).

2. Suture (10 ; 110) selon la revendication 1, dans laquelle la boucle (12 ; 112) est sensiblement tubériforme lorsque le matériau polymère à mémoire de forme est en configuration temporaire.

3. Suture (10 ; 110) selon la revendication 2, dans laquelle la boucle (12 ; 112) est sensiblement aplatie lorsque le matériau polymère à mémoire de forme passe de la configuration temporaire à la configuration permanente.

4. Suture (10 ; 110) selon l'une quelconque des revendications précédentes, dans laquelle le matériau polymère à mémoire de forme est configuré de sorte que la boucle (12 ; 112) s'aplatisse au cours de la transition d'une configuration temporaire à une configuration permanente.

5. Suture (10 ; 110) selon l'une quelconque des revendications précédentes, dans laquelle le matériau polymère à mémoire de forme est choisi dans le groupe constitué de matériaux dégradables, de matériaux non dégradables et de leurs combinaisons.

6. Suture (10 ; 110) selon la revendication 5, dans lequel le polymère à mémoire de forme comprend un matériau non dégradable choisi dans le groupe constitué des suivants : polyoléfines, polyéthylèneglycols, poly(oxydes d'éthylène), copolymères polyoléfiniques, polyoléfines fluorées, polyamides, polyamines, polyimines, polyesters, polyéthers, esters polybutyliques, polyuréthanes, polymères acryliques, polymères méthacryliques, polymères et copolymères d'halogénure de vinyle, alcools polyvinyliques, éthers polyvinyliques, poly(halogénures de vinylidène), polychlorofluoroéthylène, polyacrylonitrile, polyaryléthercétones, cétones polyvinyliques, aromatiques polyvinyliques, esters polyvinyliques, copolymères de monomères vinyliques, copolymères d'acrylonitrile-styrène, résines ABS, copolymères d'éthylène et d'acétate de vinyle, résines alkydes, polycarbonates, polyoxyméthylènes, polyphosphazines, polyimides, résines époxydes, aramides, rayonnes, spandex, silicones et leurs combinaisons.

7. Suture (10 ; 110) selon la revendication 5, dans laquelle le polymère à mémoire de forme comprend un matériau bioabsorbable choisi dans le groupe constitué des suivants : polyesters aliphatiques, polyamides, polyamines, poly(oxalates d'alkylène), poly(anhydrides), polyamidoesters, copoly(étheresters), poly(carbonates), poly(hydroxyalcanoates), poly (carbonates d'imide), polyimino-carbonates, polyorthoesters, polyoxaesters, polyphosphazènes, poly(fumarates de propylène), polyuréthanes, médicaments polymères, polymères, copolymères et homopolymères bioabsorbables biologiquement modifiés et leurs combinaisons.

8. Suture (10 ; 110) selon la revendication 5, dans laquelle le polymère à mémoire de forme comprend un polyester aliphatique choisi dans le groupe constitué des homopolymères et des copolymères des composés suivants : lactide, glycolide, epsilon-caprolactone, p-dioxanone, carbonate de triméthylène, dérivés alkyliques du carbonate de triméthylène, Δ-valérolactone, β-butyrolactone, γ-butyrolactone, ε-décalactone, hydroxybutyrate, hydroxyvalérate, 1,4-dioxépan-2-one, 1,5-dioxépan-2-one, 6.6-diméthyl-1,4-dioxan-2-one, 2,5-dicétomorpholine, pivalolactone, α,α-diéthylpropiolactone, carbonate d'éthylène, oxalate d'éthylène, 3-méthyl-1,4-dioxan-2,5-dione, 3,3-diéthyl-1,4-dioxan-2,5-dione, 6,8-dioxabicycloctane-7-one et leurs combinaisons.

9. Suture (10 ; 110) selon la revendication 5, dans laquelle le polymère à mémoire de forme comprend un polymère biodégradable choisi dans le groupe constitué des suivants : poly(acides aminés), collagène, élastine, fibrine, fibrinogène, soie, albumine, peptides comprenant des séquences pour la laminine et la fibronectine, acide hyaluronique, dextrane, alginate, chitine, chitosane, cellulose, glycosaminoglycane, boyau, méthylcellulose, éthylcellulose, hydroxypropylcellulose, hydroxypropylméthylcellulose, hydroxybutylméthylcellulose, acétate de cellulose, propionate de cellulose, acétate butyrate de cellulose, acétate phtalate de cellulose, carboxyméthylcellulose, triacétate de cellulose, sel sodique de sulfate de cellulose, nitrocelluloses, chitosane et leurs combinaisons.

10. Suture (10 ; 110) selon la revendication 5, dans laquelle le polymère à mémoire de forme comprend un polymère choisi dans le groupe constitué des diméthacrylates d'oligo(epsilon-caprolactone), des acrylates d'oligo(epsilon-caprolactone)butyle, de l'acrylate de n-butyle, des copolymères d'oligo(epsilon-caprolactone)diol et d'oligo(p-dioxanone)diol, de mélanges de diméthacrylate polycaprolactone et de poly(acrylate de butyle) et de leurs combinaisons.

11. Suture (10 ; 110) selon la revendication 5, dans laquelle le polymère à mémoire de forme comprend un copolymère séquencé de polydioxanone et de polylactide ; de préférence, dans laquelle le polydioxanone est présent en quantité d'environ 5 % en mole à environ 20 % en mole du copolymère et le polylactide est présent en quantité d'environ 80 % en mole à environ 95 % en mole du copolymère.

12. Suture (10 ; 110) selon la revendication 5 dans laquelle le polymère à mémoire de forme comprend un copolymère séquencé de carbonate de triméthylène et de polylactide ; de préférence, dans laquelle le carbonate de triméthylène est présent en quantité d'environ 5 % en mole à environ 20 % en mole du copolymère et le polylactide est présent en quantité d'environ 80 % en mole à environ 85 % en mole du copolymère.

13. Suture (10) selon l'une quelconque des revendications précédentes, comprenant en outre une pluralité de caractéristiques de surface (3) choisies dans le groupe constitué de barbillons, de crochets, d'encoches, de saillies, de feuilles, de dents et de leurs combinaison ; de préférence, dans laquelle la caractéristique de surface (3) comprend un polymère à mémoire de forme.
